# EUROPÄISCHE PATENTSCHRIFT

(11) **EP 1 790 383 B1**
(45) Veröffentlichungstag und Bekanntmachung des Hinweises auf die Patenterteilung: **28.07.2010**
(21) Anmeldenummer: 06023778.1
(22) Anmeldetag: 16.11.2006
(51) Int. Cl.: A61N 5/06, A61F 9/01

(54) **Verfahren und Vorrichtung zum Bearbeiten eines Werkstücks**
Method and device for machining of a workpiece
Procédé et dispositif d'usinage d'une pièce

(30) Priorität: 29.11.2005 DE 102005056958
(43) Veröffentlichungstag der Anmeldung: 30.05.2007
(73) Patentinhaber: Rowiak GmbH, 30419 Hannover (DE)
(72) Erfinder: Lubatschowski, Holger, Dr., 30989 Gehrden (DE)
(74) Vertreter: Grünecker, Kinkeldey, Stockmair & Schwanhäusser Anwaltssozietät

(56) Entgegenhaltungen:
- WO-A-93/14430
- WO-A-2005/074848
- JP-A- 2004 113 322
- US-A1- 4 461 294
- US-A1- 5 092 864
- US-A1- 2005 149 006

## Beschreibung

Die vorliegende Erfindung bezieht sich auf ein Verfahren zum Bearbeiten eines Werkstücks gemäß dem Oberbegriff des Anspruchs 1, sowie auf eine dafür einsetzbare Vorrichtung.

Ein gattungsgemäßes Verfahren und eine entsprechende Vorrichtung sind aus der WO 2005/074848 A1 und dem Artikel "Riboflavin/Ultraviolet-A-induced Collagen Crosslinking for the Treatment of Keratoconus", G. Wollensak et al., American Journal of Ophthalmology, May 2003, S. 620, bekannt. Das Verfahren und die Vorrichtung werden dort zur Behandlung des Keratokonus eingesetzt. Ein Keratokonus ist eine Erkrankung des Auges, bei der sich die Kornea, d.h. die Hornhaut des Auges, ausdünnt und an Festigkeit verliert. Unter dem Einfluss des Augen-Innendrucks führt die Schwächung der Kornea dazu, dass diese sich nach außen wölbt, was wiederum zu einer Fehlsichtigkeit des betroffenen Auges führt. Da es sich beim Keratokonus um eine fortschreitende Krankheit handelt, ist die Gefahr groß, dass sich ohne eine Behandlung der Krankheit die Fehlsichtigkeit vergrößert.

In den oben genannten Dokumenten werden ein Verfahren und eine Vorrichtung zur Behandlung eines Keratokonus vorgeschlagen. Dahinter steht der Gedanke, durch eine Vernetzung der Kollagenfibrillen in der Kornea, das so genannte "crosslinking", die Festigkeit der Kornea zu erhöhen, so dass sich diese dem Augen-Innendruck besser widersetzen kann. Zu diesem Zweck wird zunächst ein Fotosensibilisator auf das Auge appliziert, insbesondere Riboflavin bzw. eine Riboflavin-Lösung. Ein Fotosensibilisator ist dabei allgemein ein Stoff, der unter dem Einwirken von Photonen in der Lage ist, chemisch mit dem ihn aufnehmenden Material in Wechselwirkung zu treten oder in dem Material eine chemische Veränderung auszulösen, beispielsweise ein Vernetzen von Molekülen und/oder ein Verhärten des Materials. Nachdem das UV-empfindliche Riboflavin in das Auge eingezogen ist, wird das Auge einer Bestrahlung mit UV-Strahlung ausgesetzt. Als UV-Lichtquelle dienen dabei entweder eine große UV-Lampe oder ein Feld von kleineren Lichtquellen. Ausdrückliches Ziel ist es in jedem Fall, die Vorderseite der gesamten Hornhaut homogen zu bestrahlen, um die Hornhaut auch homogen zu verfestigen. Unter dem Einfluss der UV-Strahlung bewirkt der Fotosensibilisator eine Vernetzung der Kollagenfibrillen, wodurch die biomechanische Festigkeit der Hornhaut gesteigert wird, so dass diese sich unter der Wirkung des Augen-Innendrucks weniger stark verformt.

Dieses Verfahren ist für den Patienten nicht ohne Risiko. In dem oben erwähnten Artikel "Riboflavin/Ultraviolet-A-induced Collagen Crosslinking for the Treatment of Keratoconus" von G. Wollensak et al. wird darauf hingewiesen, dass es zu einer Schädigung des Auges kommen kann, wenn zu viel UV-Strahlung die Augenlinse oder die Netzhaut erreicht. Indem jedoch die UV-Strahlung von der Oberfläche der Kornea in diese eindringt, steht bei dem bekannten Verfahren die gesamte Hornhautdicke zur Absorption der UV-Strahlung zur Verfügung. Es wird daher davon ausgegangen, dass das UV-Licht stark genug abgeschwächt ist, bevor es auf die inneren bzw. hinteren Teile des Auges auftrifft.

Nachteilig an dem bekannten Verfahren ist unter anderem die zellschädigende, zytotoxische Wirkung der UV-Strahlung. Für den Patienten ist es darüber hinaus unangenehm, dass die UV-Bestrahlung pro Auge etwa eine halbe Stunde dauern soll.

Ein anderes Verfahren, das vermutlich ebenfalls eine lange Behandlungsdauer für den Patienten mit sich bringt, geht aus der US 2005/0149006 A1 hervor. Im Unterschied zur WO 2005/074848 A1 wird dabei die Hornhaut des Auges zunächst "weichgekocht", indem sie erhitzt wird und indem im Inneren der Hornhaut durch fokussiertes Laserlicht Material zerstört wird. Es kann davon ausgegangen werden, dass diese Zerstörungen die Sehfähigkeit des Patienten beeinträchtigen werden. Im Anschluss an das Aufweichen der Hornhaut wird diese in eine gewünschte Form gebracht, mit einem Fotosensibilisator versehen und - wie aus der WO 2005/074848 A1 bekannt - von der Vorderseite her großflächig mit einer UV-Lampe bestrahlt, um das vorhergehende Aufweichen der Hornhaut wieder rückgängig zu machen. Die zerstörten Stellen verbleiben dabei in der Hornhaut.

Aus der JP 2004113322 A geht ferner ein Behandlungs-und Diagnosegerät hervor, bei dem eine 2-Photonen-Anregung dazu eingesetzt wird, Bilder vom Augenhintergrund zu erhalten.

Aufgabe der vorliegenden Erfindung ist es, das aus der WO 2005/074848 A1 bekannte Verfahren dahingehend zu verbessern, dass es schneller und sicherer durchgeführt werden kann. Ferner soll auch eine Vorrichtung zur Durchführung des Verfahrens zur Verfügung gestellt werden.

Diese Aufgabe wird gelöst durch ein Verfahren mit den Merkmalen des Anspruchs 1 und durch eine Vorrichtung mit den Merkmalen des Anspruchs 13. Vorteilhafte Weiterbildungen der Erfindung sind in den Unteransprüchen angegeben.

Gemäß der vorliegenden Erfindung wird auf das transparente, wenigstens teilweise flüssigkeitsaufnehmende Werkstück ein flüssiger Fotosensibilisator appliziert, der in das Werkstück einziehen kann, bevor das Werkstück mit gepulster und fokussierter Laserstrahlung bestrahlt wird. Unter "wenigstens teilweise flüssigkeitsaufnehmend" ist gemeint, dass das Werkstück durchaus auch Bereiche aufweisen kann, die keine oder nur sehr wenig Flüssigkeit aufnehmen können. Die Laserstrahlung und der Fotosensibilisator sind so aufeinander abgestimmt, dass eine Wellenlänge des Lasers bei etwa der doppelten Wellenlänge des Absorptionsmaximums des Fotosensibilisators liegt, z.B. mit einer Abweichung von +/- 10% vom Doppelten des Absorptionsmaximums. Denkbar wäre auch eine Variante der Erfindung, bei der eine Wellenlänge des Lasers innerhalb entsprechender Abweichungen bei etwa der dreifachen, vierfachen oder n-fachen Wellenlänge des Absorptionsmaximums des Fotosensibilisators liegt.

Bei der Erfindung entsteht durch das Pulsen der Laserstrahlung in Verbindung mit der räumlichen Fokussierung bereits bei sehr geringen und das Werkstück nicht schädigenden Dosen oder mittleren Leistungen des Lasers eine so hohe Intensität, dass die Wahrscheinlichkeit für Zwei-, Drei- oder Mehrphotonenprozesse am Fokus stark ansteigt. Durch diese Prozesse wird der Fotosensibilisator aktiviert, woraufhin er eine Änderung der Materialeigenschaften des Werkstücks bewirkt, insbesondere ein Verfestigen.

Ein Vorteil der Erfindung besteht darin, dass auf das Werkstück im Vergleich zum Absorptionsmaximum des Fotosensibilisators langwelliges Licht eingestrahlt wird, das an sich keinerlei Änderungen am Werkstück bewirkt, insbesondere keine Schädigungen. Die Materialänderung ist ausschließlich auf den Fokus des Lasers beschränkt. Außerhalb des Fokusvolumens fällt die Intensität so weit ab, dass eine Aktivierung des Fotosensibilisators nicht mehr oder zumindest kaum noch stattfindet. Ein weiterer Vorteil besteht darin, dass über die Wahl der Parameter der Laserstrahlung und der Fokussierung die Größe des Fokusvolumens einstellbar und änderbar ist. Auf diese Weise kann das Volumen variiert werden, innerhalb dessen die Materialeigenschaften des Werkstücks verändert werden.

Das erfindungsgemäße Verfahren kann auf extrahierte oder künstlich erzeugte Proben biologischen oder organischen Materials, auf Pflanzen oder auch auf geeignete Kunststoffe angewendet werden. Pflanzen oder Proben biologischen Materials könnten so z.B. gezielt in bestimmten Bereichen verfestigt werden, um sie besser untersuchen zu können oder um ihr Wachstum zu verbessern. Geschützt werden soll das Verfahren selbstverständlich nur insoweit, als es nicht dem Schutzausschluss für therapeutische oder chirurgische Verfahren unterliegt.

Den im oben genannten Stand der Technik beschriebenen Kriterien, insbesondere der Forderung nach einer möglichst homogenen Bestrahlung der Kornea und nach einer Bestrahlung nur auf der Oberfläche des Werkstücks, dort des Auges, ist die vorliegende Erfindung genau entgegengesetzt. Aufgrund der Transparenz des Werkstücks für die eingesetzte Laserstrahlung kann das Laserlicht nicht nur an der Oberfläche wirken, sondern auch an jedem beliebigen anderen Ort innerhalb des Werkstücks. Dies macht das erfindungsgemäße Verfahren in seinem Einsatz sehr flexibel, da die Lage des Fokus und damit der Ort der Materialbearbeitung an die spezifischen Eigenschaften des jeweiligen Werkstücks angepasst werden können. Im Gegensatz zu einer großflächig homogenen Bestrahlung findet die Bearbeitung bei der Erfindung zunächst nur an einem räumlich eng begrenzten Ort statt. Zudem erfolgt die Bestrahlung des Materials sehr effizient, weil wegen der Transparenz des Werkstücks und der fehlenden Absorption des Laserlichtes vor dem Fokus die Strahlung weitgehend verlustfrei an den Fokus und damit an den Bearbeitungsort gebracht werden kann. Die Bearbeitung kann wegen dieser Effizienzsteigerung auch schneller durchgeführt werden.

Denkbar ist es, das Verfahren auch für nicht vollständig transparente Werkstücke einzusetzen. Allerdings kann dann eine effiziente Bearbeitung nur auf oder nahe der Oberfläche des Werkstücks erfolgen.

Weitere Vorteile können mit dem erfindungsgemäßen Verfahren erzielt werden, wenn die Lage des Fokus des Laserstrahls im oder auf dem Werkstück während der Bearbeitung verändert wird. Dazu kann entweder das Werkstück relativ zum Fokus und/oder der Ort des Fokus relativ zum Werkstück verfahren werden. Günstig ist es, wenn diese Relativbewegung zwischen zwei aufeinander folgenden Laserpulsen durchgeführt wird. Durch diese Lageänderung des Fokus sind die Bearbeitung bzw. das Verfestigen des Werkstücks nicht auf die Größe eines Fokusvolumens beschränkt, sondern es können beliebige Bereiche des Werkstücks bearbeitet werden.

Beispielsweise kann der Fokus des Laserstrahls so geführt werden, dass er insgesamt ein-, zwei-, und/oder dreidimensionale Bestrahlungsgebiete auf und/oder in dem Werkstück abtastet. Diese Bestrahlungsgebiete, an denen sich das Werkstück z.B. verfestigt, können auf beliebige Weise im Werkstück angeordnet sein. Im Gegensatz zum bekannten Verfahren ist das erfindungsgemäße Verfahren selektiv in der Bearbeitung. Daher können unter anderem sensible Bereiche des Werkstücks oder solche, an denen wegen der Art des Materials keine Verfestigung erfolgen kann, bei der Bestrahlung ausgenommen werden, was das Verfahren noch effizienter macht. Ein Bestrahlungsgebiet setzt sich jeweils aus einer Vielzahl von separat bestrahlten, nebeneinander liegenden Fokusvolumina zusammen, ist also mikroskopisch gesehen nicht homogen.

In einer Variante des Verfahrens wird der Fokus des Laserstrahls auf linienförmigen Bestrahlungsbahnen auf und/oder in dem Werkstück geführt, wobei sich wenigstens zwei dieser Bestrahlungsbahnen treffen. So wird auf oder in dem Werkstück ein beliebig geformtes Netz von Bahnen erzeugt, an denen das Werkstück verfestigt ist. Mit solch einer Netzform der Bestrahlungsgebiete kann u.U. eine größere Festigkeit erzielt werden als mit einer gleichmäßigen, homogenen Bestrahlung.

Noch schneller und effizienter wird das Verfahren, wenn die Laserstrahlung simultan an mehr als einen Ort fokussiert wird. Die Fokussierung sollte dabei möglichst so erfolgen, dass an allen Foki etwa die gleichen Bedingungen herrschen, insbesondere etwa die gleichen Intensitäten.

Vorzugsweise ist die Laserstrahlung aus dem roten und/oder infraroten Spektralbereich, beispielsweise mit Wellenlängen von 600 nm bis 1200 nm. Gerade bei Werkstücken aus biologischem Material ist dieser Spektralbereich vorteilhaft, weil die Strahlung dort keine zellschädigende Wirkung zeigt.

Für das Verfahren ist es zweckmäßig, wenn der Laser ein Kurzpuls- oder Ultrakurzpulslaser ist, da sich damit bereits bei sehr geringen Pulsenergien - verbunden mit entsprechend geringen schädigenden Nebenwirkungen - ausreichend hohe Intensitäten am Fokus erzielen lassen.

Beispielsweise können die Laserpulse Nanosekundenpulse (mit einer Dauer von 1 ns bis 1 µs), Pikosekundenpulse (mit einer Dauer von 1 ps bis 1 ns), Femtosekundenpulse (mit einer Dauer von 1 fs bis 1 ps) oder Attosekundenpulse (mit einer Dauer bis zu 1 fs) sein. Der beste Kompromiss zwischen genügend kurzen Pulsen und einem in Preis und Wartung nicht zu aufwändigen Lasersystem dürfte mit einem Femto- oder Pikosekundenlasersystem erzielt werden, z.B. mit einem Titan:Saphir- oder einem Faser-Femtosekundenlaser.

Wenn die Energie eines Laserpulses zwischen 1 pJ und mehreren 100 nJ beträgt, so lassen sich damit je nach Werkstück gute bis hervorragende Bearbeitungsergebnisse erzielen.

Zwar lässt sich das Verfahren prinzipiell bei beliebigen Repetitionsraten, also Pulswiederholungsraten, des Lasers durchführen. Im Interesse der Schnelligkeit des Verfahrens ist es jedoch günstig, wenn die Repetitionsrate des Lasers einige 100 Hz bis zu mehreren 100 MHz beträgt. Damit zwischen zwei Pulsen ggf. noch die Lage des Fokus im Werkstück verändert werden könnte, liegen ideale Repetitionsraten bei einigen MHz.

Je nach Wahl des Werkstücks und des Fotosensibilisators wird die Laserstrahlung bevorzugt derart appliziert, dass an einem Ort auf oder im Werkstück eine Energiedichte von 0,1 kJ/cm² bis zu mehreren 100 kJ/ cm² deponiert wird, vorzugsweise zwischen 10 kJ/ cm² und 150 kJ/ cm². Diese Energiedichte muss dort nicht mit einem einzigen Laserpuls deponiert werden. Vielmehr ist es auch möglich, an einen einzigen Ort nacheinander mehrere Laserpulse zu applizieren, bis dort die gewünschte Energiedichte deponiert ist.

Soll mit dem erfindungsgemäßen Verfahren z.B. biologisches Material bearbeitet werden, so kann ein Fotosensibilisator mit einem Absorptionsmaximum im Ultravioletten verwendet werden, insbesondere Riboflavin (auch Lactoflavin oder Vitamin B2). Auf Grund seines Absorptionsmaximums im Ultravioletten kann Riboflavin beim erfindungsgemäßen Verfahren mittels fokussierter roter bzw. infraroter Strahlung aktiviert werden, die das biologische Material außerhalb des Fokus nicht beeinflusst oder schädigt.

Bei einigen Anwendungen kann es vorteilhaft ein, das Werkstück während der Bestrahlung mittels eines Formgebers in eine vorbestimmte Form zu bringen. Wird das Werkstück durch die Bestrahlung verhärtet, so kann es anschließend auch ohne Hilfe des Formgebers in der vorgegebenen Form verharren.

Besonders günstig wäre es dabei für die Stabilisierung des Werkstücks in der vorbestimmten Form, wenn der Formgeber für die Laserstrahlung transparent ist und die Laserstrahlung durch den Formgeber hindurch auf oder in das Werkstück gelangt. Der Formgeber könnte dabei eine Art Kontaktlinse sein, die auf das Werkstück aufgesetzt wird und dieses während der Bestrahlung in die gewünschte Form bringt.

Die Erfindung spiegelt sich auch wider in dem Berechnen der Lage eines oder mehrerer Foki für ein vorstehend beschriebenes Verfahren. Bei dieser Berechnung wird zunächst die aktuelle Form des Werkstücks gemessen bzw. analysiert und eine davon abweichende, gewünschte Form ermittelt. In die Berechnung fließen ferner die Materialeigenschaften des Werkstücks und des Fotosensibilisators sowie ggf. die Volumenänderung des Werkstücks durch die Aufnahme des Fotosensibilisators ein, bevor die optimalen Laserparameter (z.B. die mittlere Laserleistung), die Bestrahlungsdauer und das Bestrahlungsmuster (d.h. die Lage und Abfolge der einzelnen Foki) berechnet werden.

Neben dem Bearbeitungsverfahren stellt die Erfindung auch eine Vorrichtung zum Bearbeiten eines Werkstücks zur Verfügung. Sie umfasst einen Kurzpuls- oder Ultrakurzpulslaser, ein Strahlführungssystem mit einem Fokussierelement, sowie eine Einrichtung zum Zuführen eines Fotosensibilisators an das Werkstück. Wie bereits erläutert, kann der Laser ein Nano-, Piko-, Femto- oder Attosekundenpuls-Laser sein, wobei Femto- oder Pikosekundenlaser bevorzugt werden. Das Strahlführungssystem sollte dabei so ausgebildet sein, dass der Fokus der Laserstrahlung auf oder in dem Werkstück lokalisiert ist. Die Zuführeinrichtung für den Fotosensibilisator kann eine oder mehrere Öffnungen enthalten, über die der Fotosensibilisator auf das Werkstück applizierbar ist. Denkbar wäre es auch, dass die Zuführeinrichtung eine Pumpe aufweist, z.B. eine elektrisch betriebene Pumpe.

Die erfindungsgemäße Vorrichtung kann ferner eine Positioniereinrichtung zum Positionieren des Werkstücks aufweisen, z.B. eine entsprechende Halterung, ggf. mit passenden Fixierelementen für eine sichere Befestigung des Werkstücks. Dies ist vorteilhaft, um den Fokus präzise an den gewünschten Stellen im Material platzieren zu können. Wenn die Positioniereinrichtung beweglich ist, kann damit auch eine Relativbewegung zwischen Werkstück und Fokus erzielt werden.

Vorteilhaft ist es, wenn das Strahlführungssystem eine regelbare Fokussieroptik aufweist, mittels derer die Lage des Fokus der Laserstrahlung, insbesondere die Tiefe des Fokus in dem Werkstück, regelbar ist. Mittels einer solchen Optik kann der Fokus im Werkstück schneller bewegt werden, als wenn das Werkstück verfahren wird. Die Regelung der Tiefe des Fokus im Werkstück kann z.B. durch eine Mechanik zum Bewegen der Fokussierlinse erfolgen. Für die Regelung der Tiefe kann auch ein Abstandshalter vorgesehen sein, der den Abstand zwischen der Fokussieroptik und dem Werkstück festlegt, sowie Abstandssensoren, die diesen Abstand überwachen. Für eine bezüglich der optischen Achse laterale Ablenkung des Fokus kann das Strahlführungssystem ein Scannersystem umfassen, das in der Regel zwei schwenkbare Spiegel mit senkrecht zueinander stehenden Schwenkachsen umfasst.

In einer Variante der erfindungsgemäßen Vorrichtung weist das Strahlführungssystem eine Fokussieroptik auf, die simultan mehr als einen Fokus erzeugt. Zu diesem Zweck kann beispielsweise ein Linsenarray eingesetzt werden, wobei jede Linse einen eigenen Fokus erzeugt. Die Foki können dabei z.B. auf einer planen oder gekrümmten Ebene liegen. Diese Variante der Erfindung hat den Vorteil, dass mehrere Orte im Werkstück gleichzeitig bearbeitet werden können, so dass die Bearbeitung schneller durchführbar ist. Denkbar wäre es, dass eine solche Multifokaloptik in einer Weise regelbar ist, dass die Foki gemeinsam verstellbar sind, um die gewünschten Bestrahlungsgebiete im Werkstück abfahren zu können.

Die mittlere Laserleistung beträgt je nach dem Grad der Fokussierung und der eingestellten Repetitionsrate vorzugsweise 0,5 bis 1000 mW. Um möglichst feine Strukturen auf oder in dem Werkstück bearbeiten zu können, sollte das Fokussieren unter möglichst großer Numerischer Apertur (NA) erfolgen. Je nach Arbeitsabstand und Bearbeitungsfeld könnte mit einer NA von 0,3 bis 1,4 fokussiert werden.

Um stets eine ausreichende Menge an Fotosensibilisator vorrätig zu haben, ist es zweckmäßig, wenn die Vorrichtung ein Reservoir für den Fotosensibilisator aufweist, das zudem mit dem Zuführeinrichtung in Verbindung stehen könnte.

Darüber hinaus wäre es günstig, wenn die Einrichtung zum Zuführen des Fotosensibilisators eine Dosiereinrichtung für den Fotosensibilisator aufweist, mittels derer die Dosierung des Fotosensibilisators steuerbar ist. Beispielsweise könnte die Dosiereinrichtung dafür sorgen, dass in regelmäßigen zeitlichen Abständen weitere Mengen des Fotosensibilisators auf das Werkstück abgegeben werden.

Eine Automatisierung und damit einhergehend eine weitere Effizienzsteigerung lässt sich erreichen, indem die Vorrichtung eine Steuerung zum Steuern des Lasers, der Elemente des Strahlführungssystems und/oder der Einrichtung zum Zuführen des Fotosensibilisators aufweist. Mittels der Steuerung, die einen programmierbaren Prozessor umfassen kann, können die Komponenten der Vorrichtung in optimaler Weise aufeinander abgestimmt werden.

Die Erfindung umfasst auch einen Kurzpuls- oder Ultrakurzpulslaser zur Behandlung von Keratokonus. Diese Vorrichtung könnte für eine Bearbeitung der Kornea dienen, d.h. das "Werkstück" wäre dann die Augenhornhaut (Kornea) eines Patienten. Der Laserstrahl könnte über eine spaltlampen-ähnliche Apparatur auf die Augenhornhaut geführt werden. Zweckmäßig wäre es in diesem Fall, wenn die Vorrichtung eine Einrichtung zum Stabilisieren der Position des Auges aufweist, beispielsweise einen Saugring zum Aufsetzen auf das Auge. Darüber hinaus könnte eine Liege den Komfort für den Patienten verbessern.

Ferner umfasst die Erfindung einen Kurzpuls- oder Ultrakurzpulslaser zur Behandlung von zuvor festgestellten Fehlsichtigkeiten des Auges. Auch hier wäre das "Werkstück" die Kornea eines Patienten, und auch hier könnte der Laserstrahl über eine spaltlampen-ähnliche Apparatur auf die Kornea geführt werden. Eine solche Vorrichtung fände vorzugsweise Verwendung in Verbindung mit einem Formgeber für das Auge, beispielsweise einer Kontaktlinse, um das Auge in eine sehfehlerfreie Form zu bringen, in der die Augenhornhaut dann verhärtet werden kann.

Im Folgenden wird ein bevorzugtes Ausführungsbeispiel der Erfindung anhand einer Zeichnung näher beschreben. Im Einzelnen zeigen
- Fig. 1: eine schematische Ansicht einer erfindungsgemäßen Vorrichtung,
- Fig. 2: eine schematische Ansicht eines Teils einer abgewandelten Vorrichtung und
- Fig. 3: eine perspektivische Ansicht eines mit dem erfindungsgemäßen Verfahren bearbeiteten Werkstücks.

Gleiche Teile werden in allen Figuren mit den gleichen Bezugszeichen bezeichnet.

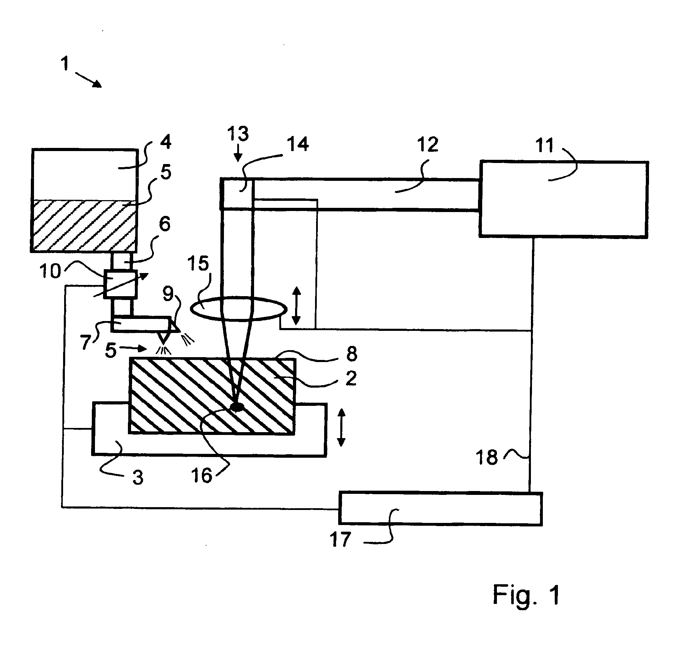

Fig. 1 zeigt eine schematische Ansicht einer erfindungsgemäßen Vorrichtung 1 zur Bearbeitung eines wenigstens teilweise flüssigkeitsaufnehmenden Werkstücks 2. Das Werkstück 2 ist in einer als Halterung ausgebildeten Positioniereinrichtung 3 gehaltert, ggf. auch durch nicht gezeigte Mittel fixiert. Die Positioniereinrichtung 3 ist in verschiedenen Raumrichtungen bewegbar - eine dieser Bewegungsmöglichkeiten ist in der Zeichnung angedeutet.

Die Vorrichtung 1 verfügt über ein Reservoir 4 zum Aufnehmen und Speichern von Fotosensibilisator 5, beispielsweise einer Riboflavin-Lösung (im Folgenden kurz: Riboflavin). Aus dem Reservoir 4 kann der Fotosensibilisator 5 über eine Zuleitung 6 an eine Zuführeinrichtung 7 fließen. Die Zuführeinrichtung 7 dient dazu, den Fotosensibilisator 5 auf geeignete Weise auf eine Oberfläche 8 des Werkstücks 2 zu applizieren. Zu diesem Zweck weist die Zuführeinrichtung 7 eine oder mehrere Zuführöffnungen 9 auf, aus denen der Fotosensibilisator 5 austreten und von dort in Form von Tröpfchen, in Form eines Sprühnebels oder in Form eines Films auf die Oberfläche 8 des Werkstücks 2 gelangen kann. In der Vorrichtung 1 in Fig. 1 sind die Zuführöffnungen 9 als Sprühdüsen ausgebildet.

In der Zuleitung 6 befindet sich zwischen dem Reservoir 4 und der Zuführeinrichtung 7 eine Dosiereinrichtung 10. Dabei kann es sich z.B. um einen Hahn oder um ein elektrisch steuerbares Ventil handeln. Die Dosiereinrichtung 10 kann sich auch unmittelbar in der Zuführeinrichtung 7 befinden. Sie dient dazu, die Zufuhr von Fotosensibilisator 5 an die Zuführöffnungen 9 und damit auf das Werkstück 2 zu regeln. Beispielsweise kann die Dosiereinrichtung 10 die Zufuhr so steuern, dass Fotosensibilisator 5 in regelmäßigen zeitlichen Abständen auf das Werkstück 2 appliziert wird.

Des weiteren umfasst die Vorrichtung 1 einen Laser 11, der gepulste Laserstrahlung 12 abgibt. Im bevorzugten Ausführungsbeispiel handelt es sich um einen Ultrakurzpulslaser, insbesondere einen Femtosekundenlaser 11 mit Pulsdauern im bereich von einigen Femtosekunden (fs) bis zu einigen 100 fs. Im Interesse möglichst geringen Wartungsaufwandes bieten sich dafür z.B. Faser-Oszillatoren an. Wegen der Kürze der Laserpulse hat die Laserstrahlung 12 ein vergleichsweise breites Spektrum. Dieses Spektrum ist so gewählt oder eingestellt, dass das Werkstück 2 bei zumindest einer mittleren Wellenlänge λ₀ des Lasers 11, vorzugsweise aber über das gesamte Spektrum des Lasers 11 weitgehend transparent ist. Das Spektrum des Lasers ist ferner so gewählt, dass es mit einer Abweichung von +/-10% bei der doppelten Wellenlänge eines Absorptionsmaximums des Fotosensibilisator 5 liegt. Je nach verwendetem Fotosensibilisator 5 kann das Spektrum des Lasers 11 daher z.B. im Bereich von 600 nm bis 1200 nm liegen, d.h. im roten oder nah-infraroten Spektralbereich.

Der Laser 11 hat eine Repetitionsrate von einigen MHz, wobei die Energie eines einzelnen Laserpulses im Bereich von Pikojoule (pJ) bis Nanojoule (nJ) liegt. Insbesondere sollte die Pulsenergie variabel einstellbar sein. Da bei der Erfindung bereits mit solchen geringen Pulsenergien gearbeitet werden kann, ist eine nachfolgende Verstärkung der Laserpulse nicht notwendig, obwohl sie durchaus vorgesehen werden könnte.

Die Strahlung 12 des Lasers 11 wird über ein Strahlführungssystem 13 an das Werkstück 2 geführt. Das Strahlführungssystem 13 umfasst dabei eine Scannereinrichtung 14 mit zwei schwenkbaren Scannerspiegeln. Über die Schwenkbewegung der Scannerspiegel ist der Laserstrahl 12 seitlich ablenkbar. Ferner umfasst das Strahlführungssystem 13 eine Fokussieroptik 15, die hier schematisch als Fokussierlinse dargestellt ist. Die Fokussieroptik 15 bündelt den Laserstrahl 12 auf einen Fokus 16. Je nach Positionierung der Fokussieroptik 15 und des Werkstücks 2 zueinander kann der Fokus 16 auf der Oberfläche 8 des Werkstücks 2 oder - wie dargestellt - im Inneren des Werkstücks 2 liegen. Die Tiefe des Fokus 16 im Werkstück 2, d.h. der Abstand des Fokus 16 von der Oberfläche 8, kann durch eine Bewegung der Positioniereinrichtung 3 und/oder durch ein Verschieben der Fokussieroptik 15 verändert werden. Die Fokussieroptik 15 ist in ihren Fokussiereigenschaften, insbesondere hinsichtlich der Lage des Fokus und der Stärke der Fokussierung, somit der Größe des Fokusvolumens, regelbar. Mittels der Fokussieroptik 15 und der Scannereinrichtung 14 kann die Lage des Fokus 16 des Lasers 11 also dreidimensional verändert werden, so dass der Fokus 16 an einen beliebigen Punkt auf oder im Werkstück 2 platziert werden kann.

Schließlich umfasst die Vorrichtung 1 auch eine Steuerung 17, beispielsweise einen programmierbaren Mikroprozessor. Über Datenleitungen 18, die die Steuerung 17 mit dem Laser 11, mit der Scannereinrichtung 14, mit der Fokussieroptik 15, mit der Positioniereinrichtung 3 und mit der Dosiereinrichtung 10 verbinden, kann die Steuerung 17 alle diese Elemente ansteuern, so dass die Vorrichtung 1 ggf. automatisch betrieben werden kann. So kann sie beispielsweise die Elemente 14, 15 des Strahlführungssystems so ansteuern, dass diese die Lage des Fokus 16 nur zwischen zwei aufeinander folgenden Laserpulsen ändern. Zudem kann sie durch eine geeignete Steuerung der Dosiereinrichtung 10 für eine regelmäßige Abgabe von Fotosensibilisator 5 auf das Werkstück sorgen.

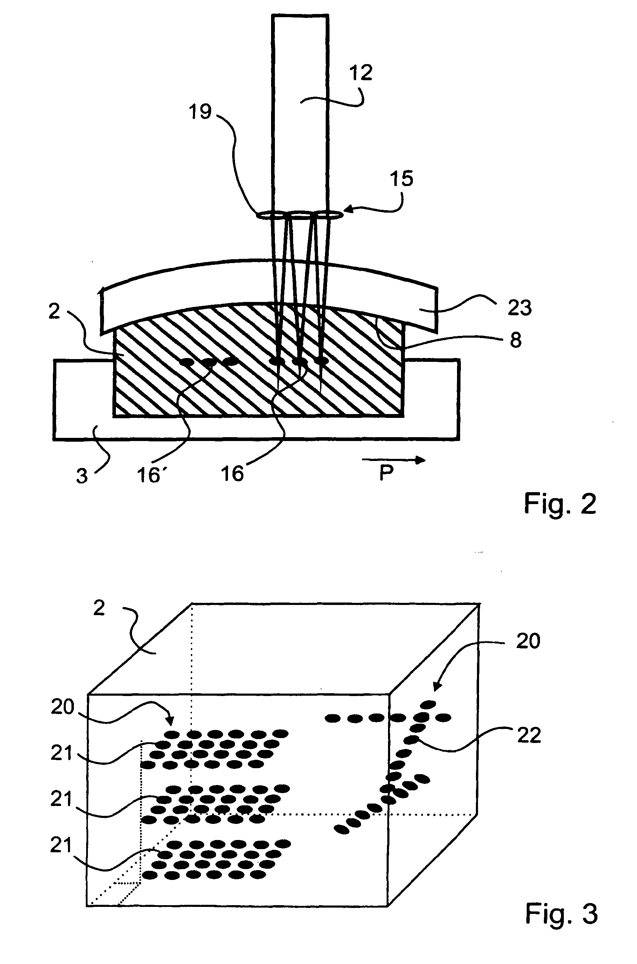

Ein Ausschnitt einer Variante der Vorrichtung 1 ist in Fig. 2 gezeigt. Im Gegensatz zu der vorstehend beschriebenen Vorrichtung 1 ist als Fokussieroptik 15 nun eine Multifokaloptik eingesetzt, d.h. eine Fokussieroptik 15, die simultan mehr als einen Fokus 16 erzeugt. Dazu kann ein Linsenarray 19 verwendet werden, von dem hier exemplarisch drei Linsen gezeigt sind. Jede der Linsen erzeugt einen eigenen Fokus 16, so dass mit einem einzigen Puls der Laserstrahlung 12 drei Foki 16 entstehen und das Werkstück simultan an drei Stellen bearbeitet wird. Auf diese Weise kann die Bearbeitungsgeschwindigkeit enorm erhöht werden.

Wird die Positioniereinrichtung 3 in Fig. 2 in der mit dem Pfeil P bezeichneten Richtung verfahren, so erzeugt der nächste Laserpuls drei neue Foki 16'. Diese Foki 16' sind hier räumlich getrennt von den ursprünglichen Foki 16 dargestellt; sie können sich jedoch auch unmittelbar an die ursprünglichen Foki 16 anschließen oder mit diesen überlappen.

Ein weiterer Unterschied gegenüber der in Fig. 1 gezeigten Vorrichtung besteht darin, dass bei der in Fig. 2 gezeigten Variante ein Formgeber 23 auf das Werkstück aufgesetzt ist. Mittels des Formgebers 23 wird die Oberfläche 8 des Werkstücks 2 in eine konvexe Form gebracht. Gemeinsam mit der Positioniereinrichtung 3 wird das Werkstück 2 auf diese Weise während der Bestrahlung in eine vorbestimmte Form gezwungen und stabil in dieser Form gehalten. Der Formgeber 23, z.B. eine harte Kontaktlinse, ist für die Laserstrahlung 12 transparent, so die Laserstrahlung 12 ohne Verluste auf und in das Werkstück 2 gelangen kann.

Das erfindungsgemäße Verfahren kann mit der Vorrichtung 1 so durchgeführt werden, dass zunächst das Werkstück 2 in der Positioniereinrichtung 3 positioniert und ggf. befestigt wird. Anschließen wird über die Zuführeinrichtung 7 Fotosensibilisator 5 auf das Werkstück 2 appliziert, z.B. in Form von Tropfen. Der Fotosensibilisator 5 zieht in das Werkstück 2 ein und dringt dabei auch in tiefere Schichten der Werkstücks 2 vor. Nach einer gewissen Wartezeit, in der dem Fotosensibilisator 5 Zeit zum Einziehen in das Werkstück 2 gegeben wird, beginnt die Bestrahlung des Werkstücks 2 mit dem Laser 11. Durch die Fokussierung der kurzen Laserpulse wird am Ort des Fokus 16 die Intensität der Strahlung 12 so hoch, dass der Fotosensibilisator 5 dort aktiviert wird. Je nach Wahl der chemischen Eigenschaften des Sensibilisators kann er am Fokus 16 zu einer Vernetzung, Verfestigung bzw. Verhärtung des Werkstücks 2 führen, so dass das Werkstück 2 dort stabiler wird.

Während der Dauer der Bestrahlung, vorzugsweise zwischen zwei aufeinander folgenden Laserpulsen, kann die Lage des Fokus 16 im Werkstück 2 durch eine Änderung der Fokussieroptik 15 und/oder durch ein Verstellen der Positioniereinrichtung 3 verändert werden. Nach und nach tastet der Fokus 16 des Lasers (bzw. die simultan erzeugten Foki 16') ganze Bestrahlungsgebiete 20 ab. Beispiele für solche Bestrahlungsgebiete 20 sind in Fig. 3 gezeigt.

Auf der linken Seite liegen drei ebene Bestrahlungsflächen 21 übereinander, so dass sie insgesamt wiederum ein dreidimensionales Bestrahlungsgebiet 20 bilden. Jede der Bestrahlungsflächen 21 ist ihrerseits aus einer Vielzahl bestrahlter Fokalbereiche zusammengesetzt. Auf der rechten Seite des Werkstücks 2 befinden sich drei linienförmige Bestrahlungsbahnen 22, die ebenfalls jeweils aus einer Vielzahl bestrahlter Fokalbereiche zusammengesetzt sind. Die Bestrahlungsbahnen 22 kreuzen bzw. überlappen sich paarweise und bilden so insgesamt ein weiteres Bestrahlungsgebiet 20. Zwischen den Bahnen 22 befinden sich jeweils unbestrahlte bzw. unbearbeitete Gebiete des Werkstücks 2. Die Bestrahlungsbahnen 22 können in beliebiger Weise auf oder im Werkstück 2 angeordnet sein. Beispielsweise können sie sich so miteinander kreuzen, dass sich insgesamt eine netzartige Anordnung von Bestrahlungsbahnen 22 bildet.

Indem die Bestrahlung des Werkstücks 2 nicht auf einen einzigen Fokusbereich 16 beschränkt bleibt, sondern über ein-, zwei- oder dreidimensionale Bestrahlungsgebiete 20 vorgenommen wird, erfolgt ein Verfestigen des Werkstücks 2 über weite Gebiete, so dass schließlich auch die Festigkeit des Werkstücks 2 insgesamt deutlich erhöht wird.

Ausgehend von dem dargestellten Ausführungsbeispiel kann die Erfindung auf vielfache Weise abgewandelt werden. So können prinzipiell beliebige gepulste Laser 11 eingesetzt werden, mit oder ohne Verstärkung der Pulse. Denkbar wäre es, einen in seinem Spektrum abstimmbaren Laser zu verwenden, um den Laser 11 auf eine Wellenlänge einstellen zu können, bei der die Aktivierung des Fotosensibilisators 5 besonders effizient ist. Ferner könnten eine Reihe von Sensoren vorgesehen sein, um das Verfahren zu überwachen, beispielsweise Sensoren zum Messen der Eigenschaften des Lasers, der Dosiervorrichtung oder der Lage des Fokus. Zweckmäßig wäre es, wenn solche Sensoren dann mit der Steuerung 17 verbunden wären, um die Steuerung des Verfahrens beeinflussen zu können.

## Patentansprüche

1. Verfahren zum Bearbeiten eines wenigstens teilweise flüssigkeitsaufnehmenden und in wenigstens einem Spektralbereich transparenten, extrahierte oder künstliche Proben biologischen oder organischen Materials, Pflanzen oder geeignete Kunststoffe umfassenden Werkstücks (2), wobei das Werkstück (2) mit gepulster und fokussierter Laserstrahlung (12) bestrahlt wird, das Spektrum der Laserstrahlung (12) wenigstens eine Wellenlänge (λ₀) im transparenten Spektralbereich des Werkstücks (2) aufweist und der Fokus (16) der Laserstrahlung (12) auf oder in dem Werkstück (2) liegt, wobei ferner vor und/oder während der Bestrahlung ein flüssiger Fotosensibilisator (5) auf das Werkstück (2) appliziert wird und in das Werkstück (2) eindringt, **dadurch gekennzeichnet, dass** der Fotosensibilisator (5) ein Absorptionsmaximum bei oder nahe der halben Wellenlänge der Laserstrahlung (12) aufweist und bei Bestrahlung ein Verhärten des Werkstücks (2) bewirkt.

2. Verfahren nach Anspruch 1, **dadurch gekennzeichnet, dass** die Parameter der Laserstrahlung (12) und der Fokussierung so eingestellt sind, dass die Aktivierung des Fotosensibilisators (5) auf den Fokus (16) der Laserstrahlung (12) beschränkt ist.

3. Verfahren nach Anspruch 1 oder 2, **dadurch gekennzeichnet, dass** die Lage des Fokus (16) des Laserstrahls (12) im oder auf dem Werkstück (2) während des Verfahrens verändert wird.

4. Verfahren nach einem der vorangehenden Ansprüche, **dadurch gekennzeichnet, dass** der Fokus (16) des Laserstrahls (12) so geführt wird, dass er insgesamt ein-, zwei-, und/oder dreidimensionale Bestrahlungsgebiete (20, 21, 22) auf und/oder in dem Werkstück (2) abtastet.

5. Verfahren nach einem der Ansprüche 1 bis 3, **dadurch gekennzeichnet, dass** der Fokus (16) des Laserstrahls (12) auf linienförmigen Bestrahlungsbahnen (22) auf und/oder in dem Werkstück (2) geführt wird und sich wenigstens zwei dieser Bestrahlungsbahnen (22) treffen.

6. Verfahren nach einem der vorangehenden Ansprüche, **dadurch gekennzeichnet, dass** die Laserstrahlung (12) simultan an mehr als einen Ort (16) fokussiert wird.

7. Verfahren nach einem der vorangehenden Ansprüche, **dadurch gekennzeichnet, dass** die Laserpulse Nano-, Piko-, Femto- oder Attosekundenpulse sind.

8. Verfahren nach einem der vorangehenden Ansprüche, **dadurch gekennzeichnet, dass** die Energie eines Laserpulses zwischen 1 pJ und mehreren 100 nJ beträgt.

9. Verfahren nach einem der vorangehenden Ansprüche, **dadurch gekennzeichnet, dass** die Repetitionsrate des Lasers (11) einige 100 Hz bis zu mehreren 100 MHz beträgt.

10. Verfahren nach einem der vorangehenden Ansprüche, **dadurch gekennzeichnet, dass** die Laserstrahlung (12) derart appliziert wird, dass an einem Ort auf oder im Werkstück (2) eine Energiedichte von 0,1 kJ/cm² bis zu mehreren 100 kJ/ cm² deponiert wird, vorzugsweise zwischen 10 kJ/ cm² und 150 kJ/ cm².

11. Verfahren nach einem der vorangehenden Ansprüche, **dadurch gekennzeichnet, dass** ein Fotosensibilisator (5) mit einem Absorptionsmaximum im Ultravioletten verwendet wird, insbesondere Riboflavin.

12. Verfahren nach einem der vorangehenden Ansprüche, **dadurch gekennzeichnet, dass** das Werkstück (2) während der Bestrahlung mittels eines Formgebers (23) in eine vorbestimmte Form gebracht wird.

13. Vorrichtung (1) zum Bearbeiten eines Werkstücks (2), insbesondere zum Durchführen eines Verfahrens nach einem der vorangehenden Ansprüche, mit einem Kurzpuls- oder Ultrakurzpulslaser (11), einem Strahlführungssystem (13), das ein Fokussierelement (15) umfasst, sowie mit einer Einrichtung (7) zum Zuführen eines Fotosensibilisators (5) an das Werkstück (2), **dadurch gekennzeichnet, dass** die Energie eines Laserpulses zwischen 1 pJ und mehreren 100 nJ beträgt.

14. Vorrichtung nach Anspruch,13 **dadurch gekennzeichnet, dass** das Strahlführungssystem (13) eine regelbare Fokussieroptik (15) aufweist, mittels derer die Lage des Fokus (16) der Laserstrahlung (12), insbesondere die Tiefe des Fokus (16) in dem Werkstück (2), regelbar ist.

15. Vorrichtung nach einem der Ansprüche13 oder 14, **dadurch gekennzeichnet, dass** das Strahlführungssystem (13) eine Fokussieroptik (15) aufweist, die simultan mehr als einen Fokus erzeugt.

16. Vorrichtung nach einem der Ansprüche13 bis 15, **dadurch gekennzeichnet, dass** die mittlere Laserleistung 0,5 bis 1000 mW beträgt.

17. Vorrichtung nach einem der Ansprüche13 bis 16, **dadurch gekennzeichnet, dass** die Einrichtung (7) zum Zuführen des Fotosensibilisators (5) eine Dosiereinrichtung (10) für den Fotosensibilisator (5) aufweist.

18. Vorrichtung nach einem der Ansprüche13 bis 17, **dadurch gekennzeichnet, dass** sie einen für die Laserstrahlung (12) transparenten Formgeber (23) aufweist, um das Werkstück (2) während der Bestrahlung in eine vorbestimmte Form zu bringen.

## Claims

1. Method for processing an at least partially fluid-absorbent and in at least one spectral region transparent workpiece (2), the workpiece (2) comprising extracted or artificial samples of a biological or organic material, plants or suitable plastics material, wherein the workpiece (2) is irradiated with pulsed and focussed laser radiation (12), the spectral range of the laser radiation (12) comprises at least one wavelength (λ₀) in the transparent spectral region of the workpiece (2), the focus (16) of the laser radiation (12) is situated on or within the workpiece (2), and before and/or during the irradiation a fluid photo-sensitizer (5) is applied onto the workpiece (2) and ingresses into the workpiece (2), **characterised in that** the photo-sensitizer (5) comprises an absorption peak at or near half said at least one wavelength of the laser radiation (12) and causes a hardening of the workpiece (2) upon being irradiated.

2. Method according to claim 1, **characterised in that** the parameters of the laser radiation (12) and of the focussing are chosen such that the activation of the photo-sensitizer (5) is confined to the focal region (16) of the laser radiation (12).

3. Method according to claim 1 or 2, **characterised in that** the position of the focus (16) of the laser radiation (12) on or within the workpiece (2) is variable during the processing.

4. Method according to any of the preceding claims, **characterised in that** the focus (16) of the laser radiation (12) is guided in such a way that in total it scans one-dimensional, two-dimensional and/or three-dimensional irradiation zones (20, 21, 22) on and/or within the workpiece (2).

5. Method according to any of claims 1 to 3, **characterised in that** the focus (16) of the laser radiation (12) is guided in such a way that it scans line-shaped irradiation zones (22) on and/or within the workpiece (2), and at least two of these line-shaped irradiation zones (22) intersect.

6. Method according to any of the preceding claims, **characterised in that** the laser radiation (12) is focussed simultaneously onto more than one locus (16).

7. Method according to any of the preceding claims, **characterised in that** the laser pulses are nanosecond pulses, picosecond pulses, femtosecond pulses or attosecond pulses.

8. Method according to any of the preceding claims, **characterised in that** the energy of one laser pulse is at or between 1 pJ and several 100 nJ.

9. Method according to any of the preceding claims, **characterised in that** the repetition rate of the laser (11) is at or between 100 Hz and several 100 MHz.

10. Method according to any of the preceding claims, **characterised in that** the laser radiation (12) is applied in such a way that on one locus on or within the workpiece (2) an energy density of 0.1 kJ/cm² up to several 100 kJ/cm² is deposited, preferably between 10 kJ/cm² and 150 kJ/cm².

11. Method according to any of the preceding claims, **characterised in that** a photo-sensitizer (5) with an absorption peak in the ultraviolet spectral region is used, in particular Riboflavin.

12. Method according to any of the preceding claims, **characterised in that** the workpiece (2) is maintained in a predetermined shape by a shaping member (23) during the processing.

13. Device (1) for processing a workpiece (2), in particular for performing a method according to any of the preceding claims, comprising a short pulse laser or ultrashort pulse laser (11), a beam guiding system (13) with a focussing element (15), and a means (7) for applying a photo-sensitizer (5) onto the workpiece (2), **characterised in that** the energy of one laser pulse is 1 pJ to several 100 nJ.

14. Device according to claim 13, **characterised in that** the beam guiding system comprises (13) controllable focusing optics (15), by means of which the position of the focus (16) of the laser radiation (12) is controllable, in particular the depth of the focus (16) within the workpiece (2).

15. Device according to any of claims 13 or 14, **characterised in that** the beam guiding system (13) comprises focusing optics (15) simultaneously generating more than one focus.

16. Device according to any of claims 13 to 15, **characterised in that** the average laser power is 0.5 mW to 1000 mW.

17. Device according to any of claims 13 to 16, **characterised in that** the means (7) for applying the photo-sensitizer (5) comprises metering means (10) for the photo-sensitizer (5).

18. Device according to any of claims 13 to 17, **characterised by** comprising a shaping member (23) transparent for the laser radiation (12) and adapted to maintain the workpiece (2) in a predetermined shape during the irradiation.

## Revendications

1. Procédé pour usiner une pièce (2) qui absorbe au moins en partie le liquide, qui est transparente au moins dans une zone spectrale et qui comprend des échantillons extraits ou artificiels de matériau biologique ou organique, des plantes ou des matières plastiques appropriées, la pièce (2) étant soumise à un rayonnement laser (12) pulsé et focalisé, le spectre du rayonnement laser (12) présentant au moins une longueur d'onde (λ₀) dans la zone spectrale transparente de la pièce (2) et le point focal (16) du rayonnement laser (12) étant situé sur ou dans la pièce (2), et un photosensibilisateur liquide (5) étant appliqué sur la pièce (2) avant et/ou pendant l'exposition au rayonnement et pénétrant dans la pièce (2), **caractérisé en ce que** le photosensibilisateur (5) présente un maximum d'absorption au niveau ou près de la moitié de la longueur d'onde du rayonnement laser (12) et provoque, lors de l'exposition au rayonnement, un durcissement de la pièce (2).

2. Procédé selon la revendication 1, **caractérisé en ce que** les paramètres du rayonnement laser (12) et de la focalisation sont réglés de telle sorte que l'activation du photosensibilisateur (5) soit limitée au point focal (16) du rayonnement laser (12).

3. Procédé selon la revendication 1 ou 2, **caractérisé en ce que** la position du point focal (16) du rayon laser (12) dans ou sur la pièce (2) est modifiée pendant le procédé.

4. Procédé selon l'une des revendications précédentes, **caractérisé en ce que** le point focal (16) du rayon laser (12) est guidé de manière à balayer dans l'ensemble des zones de rayonnement à une, deux et/ou trois dimensions (20, 21, 22) sur et/ou dans la pièce (2).

5. Procédé selon l'une des revendications 1 à 3, **caractérisé en ce que** le point focal (16) du rayon laser (12) est guidé sur des trajectoires de rayonnement (22) linéaires sur et/ou dans la pièce (2) et au moins deux de ces trajectoires de rayonnement (22) se rejoignent.

6. Procédé selon l'une des revendications précédentes, **caractérisé en ce que** le rayonnement laser (12) est focalisé simultanément à plus d'un endroit (16).

7. Procédé selon l'une des revendications précédentes, **caractérisé en ce que** les impulsions lasers sont des impulsions de l'ordre de la nanoseconde, de la picoseconde, de la femtoseconde ou de l'attoseconde.

8. Procédé selon l'une des revendications précédentes, **caractérisé en ce que** l'énergie d'une impulsion laser est située entre 1 pJ et quelques 100 nJ.

9. Procédé selon l'une des revendications précédentes, **caractérisé en ce que** la vitesse de répétition du laser (11) est située entre quelques 100 Hz et quelques 100 MHz.

10. Procédé selon l'une des revendications précédentes, **caractérisé en ce que** le rayonnement laser (12) est appliqué de telle sorte qu'à un endroit situé sur ou dans la pièce (2), une densité d'énergie de 0,1 kJ/cm² à quelques 100 kJ/cm² soit déposée, de préférence entre 10 kJ/cm² et 150 kJ/cm².

11. Procédé selon l'une des revendications précédentes, **caractérisé en ce qu'**on utilise un photosensibilisateur (5) avec un maximum d'absorption dans l'ultraviolet, en particulier de la riboflavine.

12. Procédé selon l'une des revendications précédentes, **caractérisé en ce que** pendant l'exposition au rayonnement, on donne à la pièce (2) une forme prédéfinie, à l'aide d'un élément de façonnage (23).

13. Dispositif (1) pour usiner une pièce (2), en particulier pour mettre en oeuvre un procédé selon l'une des revendications précédentes, avec un laser à impulsions courtes ou ultracourtes (11), un système de guidage de faisceau (13) qui comprend un élément de focalisation (15), et un dispositif (7) pour amener un photosensibilisateur (5) sur la pièce (2), **caractérisé en ce que** l'énergie d'une impulsion laser est située entre 1 pJ et quelques 100 nJ.

14. Dispositif selon la revendication 13, **caractérisé en ce que** le système de guidage de faisceau (13) comporte une optique de focalisation réglable (15) à l'aide de laquelle la position du point focal (16) du rayonnement laser (12), en particulier la profondeur du point focal (16) dans la pièce (2), est réglable.

15. Dispositif selon l'une des revendications 13 ou 14, **caractérisé en ce que** le système de guidage de faisceau (13) comporte une optique de focalisation (15) qui génère simultanément plus d'un point focal.

16. Dispositif selon l'une des revendications 13 à 15, **caractérisé en ce que** la puissance laser moyenne est de 0,5 à 1000 mW.

17. Dispositif selon l'une des revendications 13 à 16, **caractérisé en ce que** le dispositif (7) pour amener le photosensibilisateur (5) comporte un doseur (10) pour le photosensibilisateur (5).

18. Dispositif selon l'une des revendications 13 à 17, **caractérisé en ce qu'**il comporte un élément de façonnage (23) transparent pour le rayonnement laser (12), pour donner une forme prédéfinie à la pièce (2) pendant l'exposition au rayonnement.
